# EUROPEAN PATENT APPLICATION

(11) **EP 1 452 145 A1**
(43) Date of publication of application: **01.09.2004**
(21) Application number: 04004255.8
(22) Date of filing: 25.02.2004
(51) Int. Cl.: A61B 17/80

(54) **Anatomic miniplate for osteosynthesis of the first phalange**

(30) Priority: 26.02.2003 FR 0302507
(71) Applicant: Centerpulse Orthopedics Ltd., 6340 Baar (CH); Société Civile ASTRID, 69450 Saint Cyr au Mont d'Or (FR)
(72) Inventor: Coillard-Lavirotte, Jean-Yves, 69450 Saint Cyr au Mont d'Or (FR); Jarde, Olivier, 80000 Amiens (FR); Tourne, Yves, 38610 Gieres (FR)
(74) Representative: Manitz, Finsterwald & Partner GbR

(57) **Abstract**

Miniplate designed for the osteosynthesis of a phalange Pi (first phalange), comprising firstly an anchor and positioning stud (2) at one of its ends approximately perpendicular to the plate (1) and an adjacent attachment screw, designed to cooperate with the widest proximal end (4) of the phalange (5) and secondly at least one other attachment screw passing through a compression hole (7) in the said plate and designed to cooperate with a distal end (8) of the same phalange (5), characterised in that the miniplate has an anatomic profile in its frontal plane and in its sagittal plane.

## Description

This invention relates to an anatomic miniplate for osteosynthesis of the first phalange of a right or left foot.

The main indications are:
- osteotomy for correcting hallux valgus of the first phalange P1,
- shortening osteotomy of P1,
- shortening and hallux valgus correction osteotomy of P1.

It is known that osteotomy can be done on a phalange that is deformed or causes pain or is even infected, in order to overcome this problem.

This is done by cutting the said phalange P1 at the location of the deformation and removing a wedge-shaped part at a determined angle, so as to correct the deformation by putting the two parts of the sectioned phalange end to end.

It is then known how to perform osteosynthesis of the said phalange by putting the two cutting planes into compression to facilitate bone fusion.

This compression is done in a known manner by a miniplate comprising firstly an anchor and positioning stud at one of its ends approximately perpendicular to the miniplate and an adjacent attachment screw, designed to cooperate with the widest proximal end of the phalange and secondly at least one other attachment screw passing through a compression hole in the said miniplate and designed to cooperate with a distal end of the same phalange, so as to bring the parts of the sectioned phalange towards each other and put them in compression to enable and facilitate osteosynthesis.

In this type of known miniplate, the anchor and positioning stud, the attachment screw adjacent to the stud and the other attachment screw(s) are generally arranged along the same longitudinal axis of the miniplate.

Furthermore, this type of known miniplate is formed by a plane element that is incapable of optimising the position of the stud on the phalange concerned with respect to its anatomy.

Since this type of bone is very small, this usually reduces the distance to the edges of the phalange which can be weakened by cracks, split lines, etc. being initiated.

According to a first phase of the inventive procedure, an attempt was made to make the maximum use of the available area at the widest proximal end of the phalange in which the stud and an adjacent attachment screw would be fitted, so as to move as far as possible away from the edges of the phalange, thus enabling better use of the said available surface in this zone.

This is the reasoning adopted in the invention in order to overcome the problems mentioned above.

Consequently, the invention relates to a miniplate designed for a phalangian osteosynthesis of P1 (first phalange), comprising firstly an anchor and positioning stud at one of its ends approximately perpendicular to the plate and an adjacent attachment screw designed to cooperate with the widest proximal end of the phalange and secondly at least one attachment screw passing through a compression hole in the said plate and designed to cooperate with a distal end of the same phalange, characterised in that the miniplate has an anatomic profile in its frontal plane and in its sagittal plane.

with the invention, an anatomic miniplate is obtained with good stability both in the frontal plane and in the sagittal plane, due to its anatomic profile in its two planes.

This invention also relates to the characteristics that will become clear during the following description, and which will be considered in isolation or according to any possible technical combination.

This description is a non-limitative example and gives a better understanding about how the invention can be produced with reference to the attached drawings on which:
Figure 1 is a plan view of an anatomic miniplate, designed for a phalange in a left foot according to the invention before its anchor stud is folded.
Figure 2 is a plan view of an anatomic miniplate according to the invention after its anchor stud is folded.
Figure 3 is a side view of an anatomic miniplate according to Figure 2.
Figure 4 is a view of an anatomic miniplate according to Figures 1 to 3 after placement on the sagittal side of a phalange for osteosynthesis.
Figures 5, 6 and 7 represent an anatomic miniplate seen in plan view before the anchor stud is folded, after the anchor stud is folded, and a side view, designed for use on a phalange in a right foot.

The anatomic miniplate 1 globally shown in the figures comprises firstly at one of its ends an anchor and positioning stud 2 approximately perpendicular to the said anatomic miniplate 1 and an adjacent attachment screw, not shown, passing through a hole in the said miniplate 1, the stud 2 and the attachment screw passing through the hole 3 being designed to cooperate with the widest proximal end 4 of the phalange 5.

Two other attachment screws, not shown, pass through a hole 6 and a compression hole 7 located on the same longitudinal axis of the anatomic miniplate 1 and are designed to cooperate with a distal end 8 of the phalange 5 so as to bring the said proximal end 4 and distal end 8 of the said phalange 5 closer together and to put them into compression.

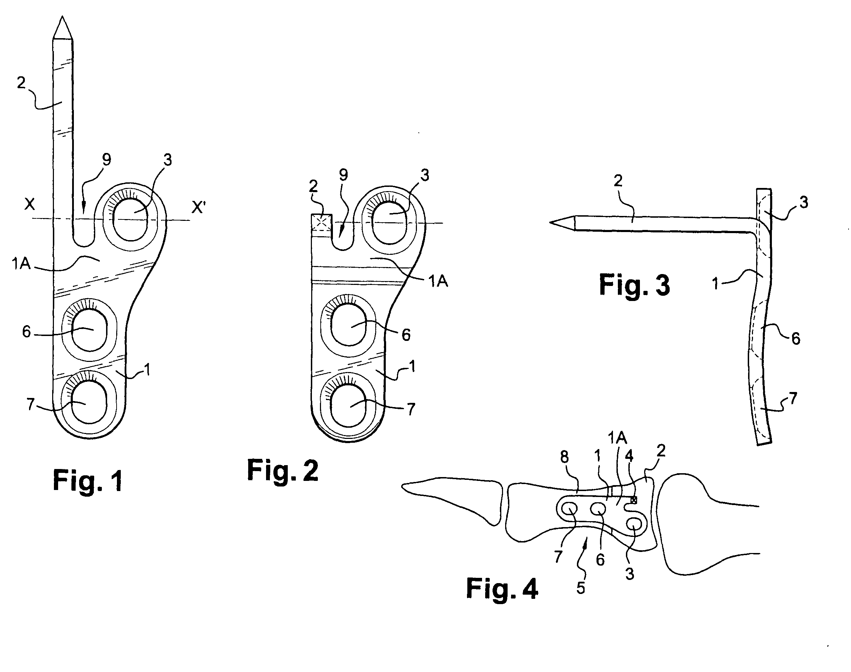

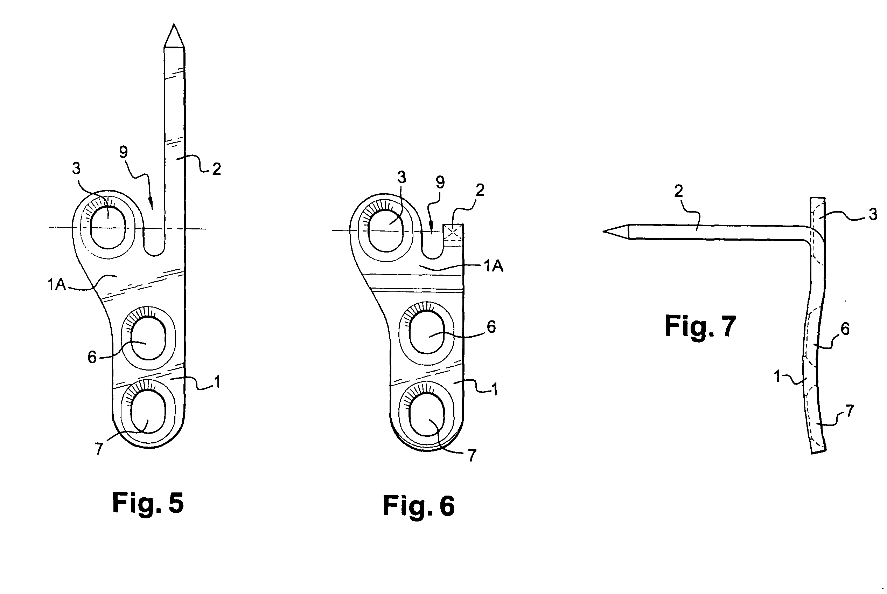

As can be seen clearly in the figures, and according to the invention, the anatomic mini-plate 1 has an anatomic profile both in its frontal plane (Figures 1, 2, 4, 5 and 6) and its sagittal plane (Figures 3 and 7).

In fact, the sagittal profile (figures 3 and 7), is significantly curved to match the corresponding profile of the phalange 5 while its frontal profile (Figures 1, 2, 4, 5 and 6) has a widened area 1A to approximately cover the widest proximal end 4 of the said phalange 5.

As can clearly be seen on the frontal views 1, 2, 4, 5 and 6, the stud 2 and the hole 3 intended for the adjacent attachment screw located in the widest proximal part 4 of the phalange 5 are positioned approximately on the same transverse axis x, x' of the phalange for better use of the available surface in this widened area 4 of the phalange 5.

According to another characteristic of the invention, the stud 2 is separated from the hole 3 through which the adjacent attachment screw will pass, by a hollowed-out part 9 formed in the said anatomic mini-plate 1 between the said stud 2 and the said attachment hole 3.

This is designed to curve the plate or curve the stud so as to better match the anatomy of the metatarsal and thus have better contact.

Preferably, the anatomic miniplate 1 forms a single-piece part obtained by cutting a metal blank according to the sagittal profile and then stamping according to the frontal profile and folding the stud 2 at an angle equal to approximately 90°.

The anatomic miniplate that has just been described may be made from stainless steel or from titanium.

As can be seen in Figures 1 to 7, the frontal and distal anatomic profiles of a left and right anatomic mini-plate correspond to a left foot phalange or a right foot phalange respectively.

## Claims

1. Miniplate designed for the osteosynthesis of a phalange P1 (first phalange), comprising firstly an anchor and positioning stud (2) at one of its ends approximately perpendicular to the plate (1) and an adjacent attachment screw, designed to cooperate with the widest proximal end (4) of the phalange (5) and secondly at least one other attachment screw passing through a compression hole (7) in the said plate and designed to cooperate with a distal end (8) of the same phalange (5), **characterised in that** the miniplate has an anatomic profile in its frontal plane and in its sagittal plane.

2. Miniplate according to claim 1, **characterised in that** the sagittal profile of the anatomic miniplate is significantly curved to match the corresponding profile of the phalange (5) while its frontal profile has a widened area (1A) to approximately cover the widest proximal end (4) of the said phalange (5).

3. Miniplate according to either claim 1 or 2, **characterised in that** the stud (2) and the hole (3) intended for the adjacent attachment screw located in the widest proximal part (4) of the phalange (5) are positioned approximately on the same transverse axis (x, x') of the phalange for better use of the available surface in this widened area (4) of the phalange (5).

4. Miniplate according to claim 3, **characterised in that** the stud (2) is separated from the hole (3) intended for the adjacent attachment screw, by a hollowed-out part (9) formed in the said anatomic miniplate (1) between the said stud (2) and the said attachment hole (3).

5. Miniplate according to one of claims 1 to 4, **characterised in that** the anatomic miniplate forms a single-piece part obtained by cutting a metal blank according to the sagittal profile and then stamping according to the frontal profile and folding the stud (2) at an angle equal to approximately 90°.

6. Miniplate according to one of the previous claims, **characterised in that** the anatomic miniplate is made from stainless steel.

7. Miniplate according to one of claims 1 to 5, **characterised in that** the anatomic miniplate is made from titanium.

8. Miniplate according to one of claims 1 to 7, **characterised in that** the frontal and distal anatomic profiles of the anatomic mini-plate correspond to a right foot phalange or a left foot phalange respectively.
